(19) Europäisches Patentamt
European Patent Office
Office européen des brevets

(11) EP 0 509 857 B1

(12) **FASCICULE DE BREVET EUROPEEN**

(45) Date de publication et mention de la délivrance du brevet: **28.02.1996 Bulletin 1996/09**

(51) Int. Cl.[6]: **C07D 277/30**, C07D 277/32, A01N 43/78

(21) Numéro de dépôt: **92400710.7**

(22) Date de dépôt: **17.03.1992**

(54) **Nouveaux dérivés de l'acide alpha-méthylène 5-thiazolacétique, leur procédé de préparation et les interdiaires de ce procédé, leur application à titre de fongicides et les compositions les renfermant**

Alphamethylen-5-Thiazoloessigsäurederivate, Zwischenprodukte und Verfahren zur Herstellung, ihre Anwendung als Fongizide und diese enthaltende Zusammensetzungen

Alpha-methylene 5-thiazolo acetic acid derivatives, intermediates and process of preparation, their use as fungicides and compositions containing them

(84) Etats contractants désignés:
**AT BE CH DE DK ES FR GB GR IT LI LU NL PT SE**

(30) Priorité: **21.03.1991 FR 9103432**

(43) Date de publication de la demande:
**21.10.1992 Bulletin 1992/43**

(73) Titulaire: **ROUSSEL UCLAF**
**F-93230 Romainville (FR)**

(72) Inventeurs:
- **Brayer, Jean-Louis**
**F-60440 Nanteuil le Haudoin (FR)**
- **Demoute, Jean-Pierre**
**F-93360 Neuilly Plaisance (FR)**
- **Mourioux, Gilles**
**F-13420 Gemenos (FR)**

(74) Mandataire: **Vieillefosse, Jean-Claude et al**
**F-93235 Romainville Cédex (FR)**

(56) Documents cités:
**EP-A- 0 299 694**
**EP-A- 0 348 766**
**EP-A- 0 402 246**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 509 857 B1

Printed by Rank Xerox (UK) Business Services
2.9.9/3.4

## Description

La présente invention concerne de nouveaux dérivés de l'acide alpha-méthylène 5-thiazolacétique, leur procédé de préparation et les intermédiaires de ce procédé, leur application à titre de fongicides et les compositions les renfermant. Certains dérivés de l'acide alpha-méthylène benzèneacétique sont décrits dans les demandes de brevet européen N° 0 299 694 et 0 348 766, comme agents fongicides. Il en est de même pour les dérivés de l'acide alpha-méthylène thiazoleacétique décrits dans la demande de brevet européen N° 0 402 246.

L'invention a pour objet les produits de formule (I) :

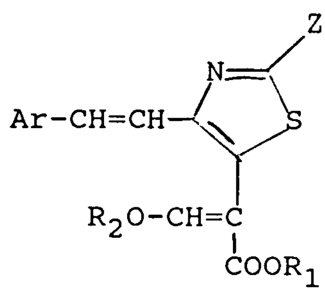

(I)

dans laquelle Ar représente un radical phényle non substitué ou substitué par un ou plusieurs radicaux identiques ou différents choisis parmi les atomes d'halogène, le radical méthylènedioxy, le radical phényle, le radical phénoxy, le radical trifluorométhyle, les radicaux alkyle linéaires ou ramifiés renfermant de un à six atomes de carbone, les radicaux alkyloxy linéaires ou ramifiés renfermant de un à six atomes de carbone ou les radicaux alkylthio linéaires ou ramifiés renfermant de un à six atomes de carbone, Z représente un atome d'hydrogène, un atome de chlore ou un radical alkyle linéaire ou ramifié renfermant de un à six atomes de carbone,

$R_1$ et $R_2$ identiques ou différents représentent indépendamment l'un de l'autre, un radical alkyle linéaire ou ramifié renfermant de un à six atomes de carbone et les doubles liaisons exocycliques sont indépendamment l'une de l'autre en configuration (Z) ou en configuration (E).

Par radical alkyle linéaire ou ramifié renfermant de un à six atomes de carbone, on peut citer le radical méthyle, éthyle, propyle, isopropyle, butyle, isobutyle, (R)-sec-butyle, (S)-sec-butyle ou tert-butyle.

Lorsque Ar représente un radical phényle substitué par un ou plusieurs atomes d'halogène, il représente notamment l'un des radicaux 2-bromo phényle, 3-bromo phényle, 4-bromo phényle, 2-chloro phényle, 3-chloro phényle, 4-chloro phényle, 2,6-dichloro phényle, 2,3,5-trichloro phényle, 2-fluoro phényle, 3-fluoro phényle, 4-fluoro phényle, 2,3-difluoro phényle, 2,4-difluoro phényle, 2,5-difluoro phényle, 2,6-difluoro phényle, 3,4-difluoro phényle, 3,5-difluoro phényle, 2,3,5,6-tétrafluoro phényle, 2,3,4,5,6-pentafluoro phényle, 2-chloro 6-fluoro phényle ou 3-chloro 4-fluoro phényle.

Lorsque Ar représente un radical phényle substitué par un ou plusieurs radicaux alkyle, il représente notamment un des radicaux ortho, méta ou para-tolyle, 2,4-diméthyl phényle, ou mésityle.

Lorsque Ar représente un radical phényle substitué par un ou plusieurs radicaux alkyloxy, il représente notamment un des radicaux 2-méthoxy phényle, 3-méthoxy phényle, 4-méthoxy phényle, 4-éthoxy phényle, 4-butoxy phényle, 2,4-diméthoxy phényle ou 3,4,5-triméthoxy phényle.

Lorsque Ar représente un radical phényle substitué par un ou plusieurs radicaux alkylthio, il représente notamment un radical 4-méthylthio phényle.

Lorsque Ar représente un radical phényle substitué par plusieurs radicaux différents, il représente notamment un des radicaux 5-bromo 2-méthoxy phényle, 3-bromo 4,5-diméthoxy phényle, 6-bromo 3,4-diméthoxy phényle ou 4-méthoxy 3-méthyl phényle.

L'invention a particulièrement pour objet les produits de formule (I) telle que définie précédemment dans laquelle $R_1$ et $R_2$ représentent un radical méthyle, ceux dans laquelle Z représente un atome d'hydrogène, un atome de chlore ou un des radicaux choisis parmi les radicaux méthyle, éthyle, propyle, isopropyle et spécialement ceux dont les noms suivent:

- le 2-isopropyl alpha-[(Z)-méthoxy méthylène] 4-[(E)-styryl] 5-thiazolacétate de méthyle,
- le alpha-[(Z)-méthoxy méthylène] 2-méthyl 4-[(E)-styryl] 5-thiazolacétate de méthyle,
- le alpha-[(Z)-méthoxy méthylène] 4-[(E)-styryl] 5-thiazolacétate de méthyle.

L'invention a également pour objet un procédé de préparation des produits de formule (I) telle que définie précédemment, caractérisé en ce qu'un ester de l'acide 5-Halo 4-oxo pentanoïque de formule (II) :

$$X\text{-}CH_2\text{-}CO\text{-}CH_2\text{-}CH_2\text{-}COOR_1 \quad (II)$$

dans laquelle X représente un atome de chlore ou de brome et $R_1$ a la signification indiquée ci-dessus, est traité par un phosphate de trialkyle de formule (III) :

$$(R_3O)_3P \qquad (III)$$

dans laquelle $R_3$ représente un radical méthyle ou un radical éthyle, pour former le phosphonate de formule (IV) :

$$(R_3O)_2\overset{O}{\overset{\uparrow}{P}}-CH_2-CO-CH_2-CH_2-COOR_1 \qquad (IV)$$

produit de formule (IV), que l'on traite en présence d'une base avec un aldéhyde de formule (V) :

$$Ar\text{-}CHO \qquad (V)$$

dans laquelle Ar a la signification indiquée ci-dessus, pour obtenir le composé de formule (VI).:

$$Ar\text{-}CH{=}CH\text{-}CO\text{-}CH_2\text{-}CH_2\text{-}COOR_1 \qquad (VI)$$

ester de formule (VI), que l'on transforme par action d'un trialkyl chloro silane de formule (VII) :

$$R_4R_5R_6SiCl \qquad (VII)$$

dans laquelle $R_4$, $R_5$ et $R_6$ identiques ou différents, représentent un radical alkyle linéaire ou ramifié renfermant de un à quatre atomes de carbone, en un éther d'énol silylé de formule (VIII) :

$$Ar\text{-}CH{=}CH\text{-}C(OSiR_4R_5R_6){=}CH\text{-}CH_2\text{-}COOR_1 \qquad (VIII)$$

produit de formule (VIII) qui, par bromation permet d'obtenir le dérivé de formule (IX) :

$$Ar\text{-}CH{=}CH\text{-}CO\text{-}CHBr\text{-}CH_2\text{-}COOR_1 \qquad (IX)$$

que l'on fait réagir soit avec le produit de formule (X) :

$$Z_1\text{-}CS\text{-}NH_2 \qquad (X)$$

dans laquelle $Z_1$ représente un atome d'hydrogène ou un radical alkyle linéaire ou ramifié, renfermant de un à six atomes de carbone, pour former un produit de formule $(XI)_1$ :

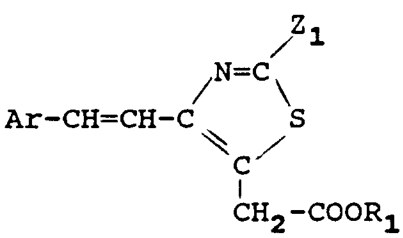

$(XI)_1$

soit avec un produit de formule (XII) :

$$Alc_1\text{-}O\text{-}CS\text{-}NH_2 \qquad (XII)$$

dans laquelle $Alc_1$ représente un radical alkyle renfermant de 1 à 4 atomes de carbone, pour former le dérivé de la 2-thiazolinone de formule (XIII) :

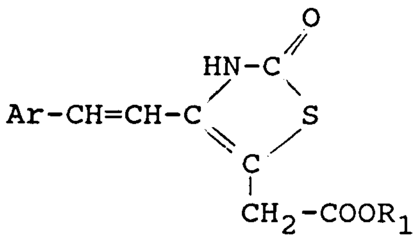

(XIII)

produit de formule (XIII) que l'on traite avec un réactif de chloration des fonctions carbonyle pour obtenir le produit de formule $(XI)_2$ :

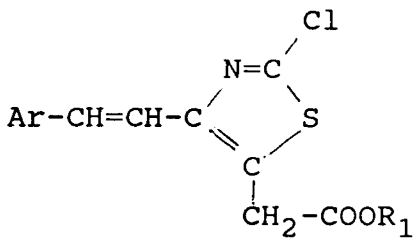

$(XI)_2$

produits de formules $(XI)_1$ ou $(XI)_2$, que l'on condense en présence d'une base avec un acétal du diméthyl formamide de formule (XVII) :

$$Me_2N\text{-}CH(OR_7)_2 \qquad (XVII)$$

dans laquelle $R_7$ représente un radical alkyle linéaire ou ramifié renfermant de un à six atomes de carbone, pour obtenir respectivement les produits de formules $(XVIII)_1$ ou $(XVIII)_2$, $(XVIII)_3$, et $(XVIII)_4$ :

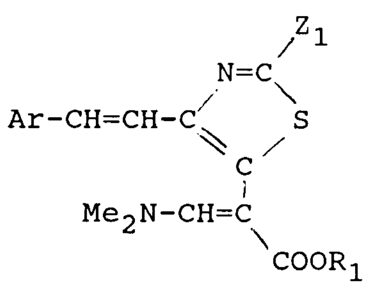

$(XVIII)_1$,

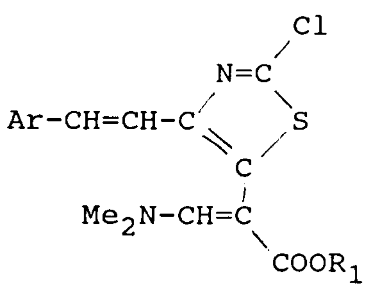

$(XVIII)_2$,

produits de formules $(XVIII)_1$ ou $(XVIII)_2$, correspondant au produit de formule (XVIII) :

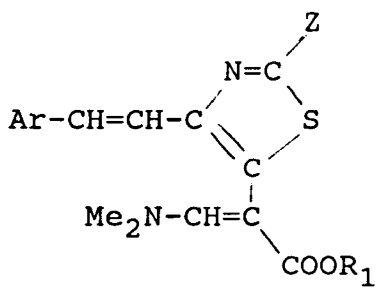

(XVIII)

dans laquelle Z, Ar et $R_1$ ont la signification indiquée ci-dessus, produit de formule (XVIII) que l'on soumet à une réaction d'hydrolyse pour obtenir le composé de formule (XIX) :

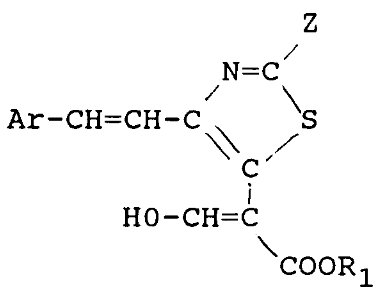

(XIX)

produit de formule (XIX) que l'on soumet à une réaction d'éthérification pour obtenir le produit de formule (I).

Dans un mode préféré d'éxécution du procédé défini ci-dessus, la réaction de préparation des produits de formule (IV) se fait dans les conditions classiques de la réaction dite d'Arbuzov, réaction chimique connue qui est décrite de manière générale dans : "Pure and Applied Chemistry", Volume 9, pages 307 à 335, (1964) ;

la préparation des produits de formule (VI) se fait dans les conditions classiques de la réaction dite d'Horner-Emmons, réaction chimique connue et décrite de manière générale dans Chemical Reviews Volume 74, Pages 87 à 99, (1974) ;

le trialkyl chlorosilane utilisé pour la synthèse de l'éther d'énol silylé de formule (VIII) est par exemple le triméthyl chloro silane ou le tert-butyl diméthyl chloro silane et la réaction est effectuée en présence d'une base azotée telle que le 1,5-diaza 5-bicyclo[5.4.0]undécène (DBU) ou le 1,5-diaza 5-bicyclo[4.3.0]nonène (DBN) ;

le produit de formule (VIII) est bromé sans purification préalable par un agent de bromation classique tel que le brome ou le N-bromo succinimide (NBS) ;

la réaction de cyclisation en produit de formule $(XI)_1$ se fait en milieu alcoolique, par exemple dans le méthanol ou l'éthanol ;

la préparation des produits de formule (XIII) se fait par exemple par action du thiocarbamate d'éthyle sur le produit de formule (IX) dans le méthanol ou l'éthanol ;

la chloration en produit de formule $(XI)_2$ se fait par exemple par action du chlorure de phosphoryle en présence d'une base azotée telle que la 2,6-lutidine ;

l'éthérification du produit de formule (XIX) est effectuée au moyen d'un halogénure d'alkyle par exemple l'iodure de méthyle;

la préparation des produits de formule (I) à partir des produits de formule (XVIII) peut être effectuée sans isoler l'intermédiaire de formule (XIX).

Les produits de formule (II) peuvent être préparés à partir de l'acide 4-oxo pentanoïque (ou acide lévulinique) qui est un produit commercial, par estérification de cet acide suivie d'une halogénation en position alpha de la fonction carbonyle comme celà est décrit plus loin dans la préparation du 5-bromo 4-oxo pentanoate de méthyle ; certains produits de formule (II) sont connus et leur préparation est décrite dans : Pichat et coll. ; Bulletin de la Société Chimique de France (1956) page 1750 ; Rappe, Ark. Kemi Volume 14, (1959) pages 467 à 469 ; Dannenberg et Laüfer, Chemisches Bericht volume 89, (1956) pages 2242 à 2252 ; Ratusky et Sorm, Collect. volume 23, (1958) pages 467 à 476. Les produits de formule (III) et la plupart des produits de formule (V) sont des produits commerciaux. Les produits de formules (IX), $(XI)_1$, $(XI)_2$, (XIII), (XVIII) et (XIX) sont nouveaux et sont aussi objet de la présente invention.

Les composés de formule (I) présentent d'intéressantes propriétés fongicides les rendant susceptibles d'être utilisés pour la protection vis-à-vis des champignons pathogènes. Il peut s'agir de la protection des plantes, de la protection des locaux ou de la protection des animaux.

Ces propriétés peuvent également être utilisées en hygiène et médecine humaine et animale.

Les composés de l'invention permettent de lutter contre de très nombreux champignons phytopathogènes, notamment contre : Erisyphe graminis, Sphaerotheca macularis, Sphaerotheca fuliginea, Podosphaera leucotricha, Uncinula necator, Helminthosporium sp., Rhynchosporium sp., Pseudocercosporella herpotrichoides et Gaeumannomyces graminis, Ustilago sp., Cercospora arachidicola et Cercosporidium personatum, Cercospora sp., Botrytis cinerea, Alternaria sp., Venturia inaequalis, Plasmopara viticola, Bremia lactucae, Peronospora sp., Pseudoperonospora humuli, Pseudoperonospora cubensis, Phytophthora sp. infestans, Phytophthora sp., Puccinia recondita, Thanatephorus cucumeris, Rhizoctonia sp. ou encore des champignons ou levures intéressant la santé humaine comme Candida albicans ou Trychophyton sp.

L'invention a donc pour objet l'utilisation à titre de fongicides des produits de formule (I) telle que définie précédemment.

L'invention a de plus pour objet les compositions fongicides comprenant à titre de principe actif, au moins un des produits de formule (I) telle que définie précédemment.

Les compositions selon l'invention sont préparées selon les procédés usuels de l'industrie agrochimique par exemple, ces compositions peuvent se présenter sous forme de poudres, granulés, suspensions, émulsions, solutions ou autres préparations employées classiquement pour l'utilisation de ce genre de composés.

Outre le principe actif, ces compositions contiennent en général, un véhicule et/ou un agent tensio-actif ionique ou non ionique, assurant une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselghur.

Les exemples suivants et les études biologiques suivantes illustrent l'invention sans toutefois la limiter.

**Exemple 1 : 2-isopropyl alpha-[(Z)-méthoxy méthylène] 4-[(E)-styryl] 5-thiazolacétate de méthyle.**

Stade A : 5-(diéthoxy phosphoryl) 4-oxo pentanoate de méthyle.

On ajoute 10,5 $cm^3$ de phosphite de triéthyle dans 12,54 g de 5-bromo 4-oxo pentanoate de méthyle (dont la préparation est décrite plus loin) chauffés a 140°C et sous agitation, laisse au reflux pendant une heure puis distille le bromure d'éthyle formé. On obtient après rectification sous pression réduite, 13,5 g d'une huile incolore. $(Eb_{0,1})$ = 155°C.

Stade B : (E)-4-oxo 6-phényl 5-hexènoate de méthyle.

Une solution de 2,66 g du phosphonate préparé au stade A dans 25 $cm^3$ de tétrahydrofuranne est ajoutée à 0°C à 0,48 g d'hydrure de sodium à 50 % dans l'huile dans 20 $cm^3$ de tétrahydrofuranne, et le mélange est agité trente minutes à 0°C, puis on ajoute 1,06 g de benzaldehyde et 15$cm^3$ de tétrahydrofuranne ; le mélange est laissé revenir à 20-21°C sous agitation pendant deux heures, puis est versé sur une solution d'acide chlorhydrique 2N. Après extraction avec du chlorure de méthylène, la phase organique est sèchée sur sulfate de sodium, filtrée et le filtrat amené à sec. Le résidu obtenu est chromatographié sur silice, en éluant par le mélange hexane-acétate d'éthyle (7-3). Après évaporation du solvant et séchage, on obtient 1,1 g de cristaux. Point de fusion = 54,5°C

Chromatographie sur couche mince ; Rf = 0,23 [éluant : hexane-acétate d'éthyle (7-3)].

| Analyse Infrarouge ($CHCl_3$) | |
|---|---|
| ester non conjugué | 1735 $cm^{-1}$ |
| cétone conjuguée | 1691 $cm^{-1}$ |
| cycle aromatique | 1497, 1578, 1612 et 1625 $cm^{-1}$ |
| double liaison en configuration E | 1667 et 989 $cm^{-1}$ |

| Analyse RMN ($CDCl_3$) ppm | |
|---|---|
| $CH_3$ | 3,67 ; |
| les $CH_2$ | 2,53 à 3,13 ; |
| Protons éthyléniques en configuration E | 6,55 et 6,82 et 7,43 et 7,70 J=16Hz ; |
| aromatiques | 7,17 à 7,67. |

Stade C : 6-phényl 4-[(triméthylsilyl) oxy] hexa-3,5-diènoate de méthyle.

On mélange 3 g du produit préparé au stade précédent, 45 $cm^3$ d'éther diéthylique et 2,2 $cm^3$ de triméthylchlorosilane. On refroidit à 0°C puis ajoute 2,5 $cm^3$ de 1,5-diaza 5-bicyclo[5.4.0]undécène (DBU) et 20 $cm^3$ d'éther diéthylique ; on agite en laissant remonter la température à 20-21°C pendant une heure. Après filtration, l'éther est évaporé et on récupère 4,2 g du produit recherché qui est utilisé tel quel au stade suivant.

Stade D : 3-bromo 4-oxo 6-phényl 5-hexènoate de méthyle.

On refroidit à 0°C, un mélange comprenant 4,2 g du produit préparé au stade précédent, 40 $cm^3$ de tétrahydrofuranne et 2,7 g de N-bromo succinimide, laisse sous agitation pendant une heure à cette température, puis amène à sec et chromatographie le résidu sur silice, en éluant par le mélange hexane-acétate d'éthyle (8-2). On recueille ainsi 1,9 g du produit attendu.

Chromatographie sur couche mince ; Rf = 0,28 [éluant : hexane-acétate d'éthyle (8-2)].

| Analyse Infrarouge : ($CHCl_3$) | |
|---|---|
| ester non conjugué | 1734 $cm^{-1}$ |
| cétone conjuguée, cycle | complexe avec pics à 1498, |
| aromatique et double liaison | 1578, 1612, 1668 et 1692 $cm^{-1}$. |

| Analyse RMN ($CDCl_3$) 60 MHz ppm | |
|---|---|
| $CH_3$ | 3,73 |
| -$\underline{CH_2}$-$COOCH_3$ | 2,78 - 2,88 et 3,06 - 3,17 |
| $\underline{CH}$Br | 4,86 - 4,97 et 4,99 - 5,10 |
| Protons éthyléniques en configuration E | 6,83 - 7,09 et 7,68 - 7,93 |
| Aromatiques | 7,28 à 7,90 |

Stade E : 2-isopropyl 4-[(E)-styryl] 5-thiazolacétate de méthyle.

On mélange 6 g du produit obtenu au stade précédent avec 60 $cm^3$ de méthanol et 2,1 g de 2-méthyl propanethioamide dont la préparation est décrite dans la demande de brevet européen publiée sous le numéro 0402246 et porte au reflux pendant cinq heures. On laisse revenir le mélange à 20-25°C agite pendant seize heures, puis amène à sec. Le résidu est chromatographié sur silice en éluant par le mélange hexane-acétate d'éthyle (8-2). On recueille ainsi après évaporation des solvants, 1,75 g du produit recherché.

Chromatographie sur couche mince ; Rf = 0,3 [éluant : hexane-acétate d'éthyle (8-2)].

| Analyse Infrarouge ($CHCl_3$) | |
|---|---|
| Ester non conjugué | 1734 $cm^{-1}$ |
| Aromatique, C=C, C=N | 1490, 1520, 1576, 1600, et 1610 $cm^{-1}$ |
| Double liaison en configuration E | 964 $cm^{-1}$ |

| Analyse RMN ($CDCl_3$, 250 MHz) | |
|---|---|
| CH-$\underline{(CH_3)_2}$ | 1,42 ppm (doublet ; J=7 Hz) ; |
| $\underline{CH}$-$(CH_3)_2$ | 3,31 ppm ; |
| $CO_2\underline{CH_3}$ | 3,75 ppm ; |
| $\underline{CH_2}$-$CO_2CH_3$ | 3,88 ppm ; |
| Proton éthylénique en position béta du radical styryle ; | 7,00 ppm (doublet ; J=16 Hz) |
| proton éthylénique en position alpha du radical styryle ; | 7,28 ppm (doublet) |
| aromatiques | 7,20 à 7,55 ppm. |

Stade F : alpha-[(diméthylamino) méthylène] 2-isopropyl 4-[(E)-styryl] 5-thiazolacétate de méthyle.

On porte à 50°C un mélange contenant 3,1 g du produit préparé au stade précédent et 30 $cm^3$ d'acétal diméthylique du diméthylformamide ; après cinq heures à cette température, on laisse revenir le milieu à 20-25°C puis maintient l'agitation pendant seize heures.Le milieu réactionnel est ensuite amené à sec et le résidu chromatographié sur silice en éluant par le mélange hexane-acétate d'éthyle (8-2). Après évaporation des solvants, on recueille 2 g du produit recherché.
Chromatographie sur couche mince ; Rf = 0,15 [éluant : hexane-acétate d'éthyle (8-2)].

| Analyse RMN ($CDCl_3$, 250 MHz) | |
|---|---|
| CH-$\underline{(CH_3)_2}$ | 1,42-1,44 ppm ; |
| N-$\underline{(CH_3)_2}$ | 2,80 ppm ; |
| $\underline{CH}$-$(CH_2)_3$ | 3,30 ppm ; |
| $CO_2\underline{CH_3}$ | 3,66 ppm ; |
| protons éthyléniques en positions alpha et béta du radical styryle ; | 6,83 ppm |
| aromatiques | 7,20 à 7,50 ppm ; |
| C=$\underline{CH}$-N | 7,76 ppm. |

Stade G : alpha-(hydroxy méthylène) 2-isopropyl 4-[(E)-styryl] 5-thiazolacétate de méthyle.

On mélange sous azote 2 g de l'ènamine préparée au stade précédent, 20 $cm^3$ de tétrahydrofuranne et 5 $cm^3$ d'une solution d'acide chlorhydrique 2N et on laisse pendant cinq heures à 20-21°C ; on évapore le tétrahydrofuranne et on utilise le produit tel quel pour le stade suivant.

Stade H : 2-isopropyl alpha-[(Z)-méthoxy méthylène] 4-[(E)-styryl] 5-thiazolacétate de méthyle.

Le produit préparé au stade précédent est dissous dans 20 $cm^3$ d'acétone puis additionné de 3 $cm^3$ d'iodure de méthyle et 4 g de carbonate de potassium et le tout est agité 16 heures à 20°C ; on amène à sec et le résidu est

chromatogaphié sur silice en éluant par le mélange hexane-acétate d'éthyle (8-2) ; on recueille 1,3 g du produit recherché.

Chromatographie sur couche mince ; Rf = 0,20 (éluant : hexane-acétate d'éthyle (8-2)].

| Analyse RMN ($CDCl_3$, 250 MHz) | |
|---|---|
| CH-<u>($CH_3$)</u>$_2$ | 1,44 ppm ; |
| <u>CH</u>-($CH_3$)$_2$ | 3,32 ppm ; |
| $CO_2$<u>$CH_3$</u> CO<u>$CH_3$</u> | 3,75 et 3,89 ppm ; |
| un des protons éthyléniques | 6,75 ppm (doublet ; J=16 Hz) ; |
| aromatiques et l'autre proton éthylénique ; | 7,12 à 7,45 ppm |
| C=<u>CH</u>-$OCH_3$ (Z) | 7,70 ppm. |

| Microanalyse : $C_{19}H_{21}NO_3S$ = 343,444 | | | | |
|---|---|---|---|---|
| calculés | C% 66,45 | H% 6,16 | N% 4,08 | S% 9,33 |
| trouvés | 66,1 | 6,1 | 4,0 | 9,3 |

<u>Exemple 2</u> : **alpha-[(Z)-méthoxy méthylène] 2-méthyl 4-[(E)-styryl] 5-thiazolacétate de méthyle.**

<u>Stade A</u> : 2-Méthyl 4-((E)-styryl] 5-thiazolacétate de méthyle.

On mélange 6 g du produit obtenu au stade D de l'exemple 1 avec 60 $cm^3$ de méthanol et 1,5 g de thioacétamide, et porte au reflux pendant trois heures. On verse ensuite sur de l'eau, neutralise avec du bicarbonate de sodium et extrait avec du chlorure de méthylène. La phase organique est séchée sur sulfate de magnésium filtrée et amenée à sec. Le résidu est chromatographié sur silice en éluant par le mélange hexaneacétate d'éthyle (7-3). On recueille ainsi après évaporation des solvants, 2,4 g du produit recherché.

Chromatographie sur couche mince ; Rf = 0,35 [éluant : hexane-acétate d'éthyle (7-3)].

| Analyse RMN ($CDCl_3$, 250 MHz) | |
|---|---|
| méthyle en position 2 | 2,70 ppm ; |
| $CH_3$ de l'ester | 3,74 ppm ; |
| <u>$CH_2$</u>-$CO_2CH_3$ | 3,75 ppm ; |
| Protons éthyléniques | 6,39 ppm (doublet ; J=15,5 Hz) et 7,50 ppm (doublet ; J=15,5 Hz) ; |
| aromatiques | 7,26 ; 7,35 et 7,52 ppm. |

<u>Stade B</u> : alpha-((Z)-méthoxy méthylène] 2-méthyl 4-[(E)-styryl] 5-thiazolacétate de méthyle.

On porte au reflux un mélange,contenant 3 g du produit préparé au stade précédent et 30 $cm^3$ d'acétal diméthylique du diméthylformamide ; après cinq heures à cette température, on laisse revenir le milieu à 25°C. Le milieu réactionnel est ensuite amené à sec, dissous dans 30 $cm^3$ de tétrahydrofuranne et 5 $cm^3$ d'une solution d'acide chlorhydrique 2N et laissé pendant trois heures à 25°C ; on ajoute 10 $cm^3$ d'eau, décante et extrait au chlorure de méthylène ; la phase organique est séchée sur sulfate de magnésium, filtrée et amenée à sec ; le résidu obtenu dissous dans 50 $cm^3$ d'acétone, additionné de 6 g de carbonate de potassium et puis de 12 $cm^3$ d'iodure de méthyle et le tout est agité seize heures à 20-25°C ; on amène à sec et le résidu est chromatogaphié sur silice en éluant par le mélange hexane-acétate d'éthyle (7-3) ; on recueille 1,8 g du produit recherché.

Chromatographie sur couche mince ; Rf = 0,20 [éluant : hexane-acétate d'éthyle (7-3)].

| Analyse RMN ($CDCl_3$, 250 MHz) | |
|---|---|
| méthyle en position 2 | 2,72 ppm ; |
| méthyle de l'ester et du radical méthoxy ; | 3,75 et 3,91 ppm |
| Protons éthyléniques | 6,75 ppm (doublet ; J=16 Hz) et 7,30 ppm (doublet ; J=16 Hz) ; |
| aromatiques | 7,19 à 7,50 ppm ; |
| Proton du radical méthylène [configuration (Z) de la double liaison]. | 7,71 ppm |

| Microanalyse : $C_{17}H_{17}NO_3S$ = 315,390 | | | | |
|---|---|---|---|---|
| calculés | C% 64,74 | H% 5,43 | N% 4,44 | S% 10,17 |
| trouvés | 63,9 | 5,3 | 4,3 | 9,8 |

**<u>Exemple 3</u> : alpha-[(Z)-méthoxy méthylène] 4-[(E)-styryl] 5-thiazolacétate de méthyle.**

<u>Stade A</u> : 4-[(E)-styryl] 5-thiazolacétate de méthyle.

On mélange 8 g du produit obtenu au stade D de l'exemple 1 avec 80 $cm^3$ de méthanol et 1,8 g de thioformamide dont la préparation est décrite dans la demande de brevet européen publiée sous le n° 0402246, et porte au reflux pendant quatre heures. On verse ensuite sur de l'eau et extrait au chlorure de méthylène. La phase organique est séchée sur sulfate de magnésium filtrée et amenée à sec. Le résidu est chromatographié sur silice en éluant par le mélange hexane-acétate d'éthyle (8-2). On recueille ainsi après évaporation des solvants, 2,5 g du produit recherché.

Chromatographie sur couche mince ; Rf = 0,19 [éluant : hexane-acétate d'éthyle (8-2)].

| Analyse RMN ($CDCl_3$, 250 MHz) | |
|---|---|
| méthyle de l'ester | 3,76 ppm ; |
| <u>$CH_2$</u>-$CO_2CH_3$ | 3,96 ppm ; |
| Protons éthyléniques et aromatiques | 7,06 ppm (doublet ; J=16 Hz) et de 7,2 à 7,7 ppm ; |
| Proton en position 2 du thiazole. | 8,75 ppm |

<u>Stade B</u> : alpha-[(Z)-méthoxy méthylène] 4-[(E)-styryl] 5-thiazolacétate de méthyle.

On porte à 50°C un mélange contenant 2,5 g du produit préparé au stade précédent et 25 $cm^3$ d'acétal diméthylique du diméthylformamide ; après quatre heures à cette température, on laisse revenir le milieu à 25°C. Le milieu réactionnel est ensuite amené à sec, dissous dans 30 $cm^3$ de tétrahydrofuranne et 5 $cm^3$ d'une solution aqueuse d'acide chlorhydrique 2N et laissé pendant deux heures à 25°C ; on décante et extrait avec du chlorure de méthylène ; la phase organique est séchée sur sulfate de magnésium, filtrée et amenée à sec ; le résidu obtenu dissous dans 30 $cm^3$ d'acétone, additionné de 4 g de carbonate de potassium et de 6 $cm^3$ d'iodure de méthyle et le tout est agité seize heures à 20-25°C ; on filtre, amène à sec et le résidu est chromatogaphié sur silice en éluant par le mélange hexane-acétate d'éthyle (7-3) ; on recueille 1,1 g du produit recherché.

Chromatographie sur couche mince ; Rf = 0,17 [éluant : hexane-acétate d'éthyle (7-3)].

| Analyse RMN ($CDCl_3$, 250 MHz) | |
|---|---|
| méthyle de l'ester et du radical méthoxy ; | 3,75 et 3,91 ppm |
| Protons éthyléniques [configuration (E)] | 6,82 ppm (doublet ; J=16 Hz) et 7,56 ppm (doublet ; J=16 Hz) ; |
| Aromatiques | 7,20 à 7,53 ppm ; |
| Proton du radical méthylène [configuration (Z) de la double liaison] ; | 7,75 ppm |
| Proton en position 2 du thiazole. | 8,83 ppm |

| Microanalyse : $C_{16}H_{15}NO_3S$ = 301,363 | | | | |
|---|---|---|---|---|
| calculés | C% 63,77 | H% 5,02 | N% 4,65 | S% 10,64 |
| trouvés | 63,2 | 5,0 | 4,3 | 10,5 |

**<u>Exemple 4</u> : 2-chloro alpha-[(Z)-méthoxy méthylène] 4-[(E)-styryl] 5-thiazolacétate de méthyle.**

<u>Stade A</u> : 2-oxo 4-[(E)-styryl] 5-thiazolacétate de méthyle.

20 g de produit préparé au stade D de l'exemple 1 sont mélangés à 200 $cm^3$ de méthanol puis on ajoute 8,5 g de thiocarbamate d'éthyle et le tout est chauffé au reflux pendant quatre heures ; le mélange est laissé revenir à 20-25°C puis est versé dans l'eau et extrait au chlorure de méthylène. La phase organique est séchée sur sulfate de sodium, filtrée et amenée à sec. Le produit obtenu est repris dans l'ether isopropylique puis essoré. On obtient 8,7 g de produit recherché. PF = 164,2°C. Chromatographie sur couche mince ; Rf = 0,11 [éluant : hexane-acétate d'éthyle (7-3)].

| Analyse RMN (DMSO, 250 MHz) | |
|---|---|
| méthyle de l'ester | 3,66 ppm ; |
| <u>$CH_2$</u>-$CO_2CH_3$ | 3,93 ppm ; |
| Protons éthyléniques et aromatiques | 7,05 ppm et de 7,3 à 7,56 ppm ; |
| Proton de NH ou OH | 11,45 ppm. |

<u>Stade B</u> : 2-chloro 4-[(E)-styryl] 5-thiazolacétate de méthyle.

6 g du produit préparé au stade précédent sont mélangés à 24 $cm^3$ d'oxychlorure de phosphore ($POCl_3$) puis on introduit goutte à goutte 2,5 $cm^3$ de 2,6-lutidine. Après chauffage au reflux pendant trois heures et demi, on laisse revenir le mélange à 20-25°C. On chasse l'excès de $POCl_3$ sous pression réduite, et verse le milieu réactionnel sur 40 $cm_3$ d'eau glacée et extrait avec du chlorure de méthylène ; la phase organique est séchée sur sulfate de sodium, filtrée et amenée à sec et le résidu est chromatogaphié sur silice en éluant par le mélange hexane-acétate d'éthyle (7-3) ; on recueille après évaporation des solvants, 6 g du produit recherché.

PF = 84,5°C.
Chromatographie sur couche mince ; Rf = 0,35 [éluant : hexane-acétate d'éthyle (7-3)].

| Analyse RMN ($CDCl_3$, 250 MHz) | |
|---|---|
| méthyle de l'ester | 3,77 ppm ; |
| $\underline{CH_2}$-$CO_2CH_3$ | 3,88 ppm ; |
| un proton éthylénique [configuration (E)] ; | 6,91 ppm (doublet ; J=16 Hz) |
| L'autre proton éthylénique les protons aromatiques. | de 7,28 à 7,55 ppm |

Stade C : 2-chloro alpha-[(diméthylamino) méthylène] 4-[(E)-styryl] 5-thiazolacétate de méthyle

On chauffe à 50°C un mélange contenant 5,9 g du produit préparé au stade précédent et 55 $cm^3$ d'acétal diméthylique du diméthylformamide ; après quatre heures à cette température, on laisse revenir le milieu à 25°C. Le milieu réactionnel est ensuite amené à sec et le résidu est chromatogaphié sur silice en éluant par le mélange hexane-acétate d'éthyle (7-3) ; on recueille 3,3 g du produit recherché.

Chromatographie sur couche mince ; Rf = 0,18 [éluant : hexane-acétate d'éthyle (7-3)].

| Analyse RMN ($CDCl_3$, 250 MHz) | |
|---|---|
| méthyle du radical diméthylamino ; | 2,86 ppm |
| Méthyle de l'ester | 3,67 ppm ; |
| un proton éthylénique [configuration (E)] ; | 6,78 ppm (doublet ; J=16 Hz) |
| L'autre proton éthylénique et les protons aromatiques. | de 7,21 à 7,51 ppm |

Stade D : 2-chloro alpha-[(Z)-méthoxy méthylène] 4-[(E)-styryl] 5-thiazolacétate de méthyle.

3 g du produit préparé au stade précédent sont mélangés à 30 $cm^3$ de tétrahydrofuranne puis 5 $cm^3$ d'une solution d'acide chlorhydrique 2N et le tout est laissé à 20-25°C pendant 5 heures. Le mélange est ensuite versé sur de l'eau et extrait au chlorure de méthylène ; la phase organique est séchée sur sulfate de magnésium, filtrée et amenée à sec ; le résidu obtenu est dissous dans 20 $cm^3$ d'acétone et additionné de 3 g de carbonate de potassium puis on ajoute 8 $cm^3$ d'iodure de méthyle ; quand la réaction est terminée, on filtre, amène à sec. Le résidu est chromatogaphié sur silice en éluant par le mélange chlorure de méthylène-hexane (6-4) ; on recueille après évaporation des solvants, 0,34 g de produit A et 0,80 g de produit B.

Produit A : Chromatographie sur couche mince ; Rf = 0,17 [éluant : Chlorure de méthylène-hexane (6-4)].

| Analyse RMN ($CDCl_3$, 250 MHz) | |
|---|---|
| Méthyles de l'ester et du méthoxy ; | 3,77 et 3,93 ppm |
| Un des protons éthyléniques [configuration (E)] ; | 6,67 ppm (doublet ; J=16 Hz) |
| L'autre proton éthylénique et les aromatiques ; | 7,20 à 7,60 ppm |
| Proton du méthylène [configuration (Z) de la double liaison]. | 7,73 ppm |

| Microanalyse : $C_{16}H_{14}ClNO_3S$ = 335,808 | | | | | |
|---|---|---|---|---|---|
| calculés | C% 57,23 | H% 4,20 | Cl% 10,56 | N% 4,17 | S% 9,55 |
| trouvés | 57,1 | 4,1 | 10,8 | 4,0 | 9,3 |

**Exemple 5 : 2-chloro alpha-[(E)-méthoxy méthylène] 4-[(E)-styryl] 5-thiazolacétate de méthyle.**

Il s'agit du produit B obtenu au stade D de l'exemple précédent. PF = 110°C.

Produit B : Chromatographie sur couche mince ; Rf = 0,11 [éluant : Chlorure de méthylène-hexane (6-4)].

| Analyse RMN ($CDCl_3$, 250 MHz) | |
|---|---|
| Méthyles de l'ester et du méthoxy ; | 3,77 et 4,00 ppm |
| Un des protons éthyléniques [configuration (E)] ; | 6,89 ppm (doublet ; J=16 Hz) |
| L'autre proton éthylénique les aromatiques ; | de 7,20 à 7,55 ppm |
| Proton du radical méthylène [configuration (E) de la double liaison]. | 6,79 ppm |

| Microanalyse : $C_{16}H_{14}ClNO_3S$ = 335,808 | | | | | |
|---|---|---|---|---|---|
| calculés | C% 57,23 | H% 4,20 | Cl% 10,56 | N% 4,17 | S% 9,55 |
| trouvés | 57,3 | 4,2 | 11,0 | 4,0 | 9,3 |

**Préparation du 5-bromo 4-oxo pentanoate de méthyle**

Stade A : 4-oxo pentanoate de méthyle

On agite pendant seize heures à 20-25°C, une solution contenant 300 g d'acide 4-oxo pentanoïque (acide lévulinique), 300 $cm^3$ de méthanol et 2 $cm^3$ d'acide sulfurique à 97 %, amène à sec et rectifie le résidu sous pression réduite. On recueille 290 g de l'ester méthylique attendu. $Eb_{16}$ = 87-88°C. Chromatographie sur couche mince ; Rf = 0,13 [éluant : hexane-acétate d'éthyle (8-2)].

Stade B : 5-bromo 4-oxo pentanoate de méthyle

100 g de l'ester préparé au stade précédent sont mélangés à 1600 $cm^3$ de méthanol et 2$cm^3$ d'acide acétique concentré et le tout est chauffé à 40°C ; on introduit alors 42 $cm^3$ de brome puis on laisse revenir la températue à 25°C sous agitation. Le mélange est amené à sec, le résidu obtenu dissous dans 100 $cm^3$ d'eau et neutralisé avec une solution aqueuse saturée de bicarbonate de soude. Après extraction à l'éther diéthylique, la phase organique est séchée sur sulfate de magnésium, filtrée et amenée à sec. Le résidu obtenu est chromatographié sur silice. On recueille 133,6 g de produit recherché.

Chromatographie sur couche mince ; Rf = 0,18 [éluant : hexane-acétate d'éthyle (8-2)].

## ETUDE BIOLOGIQUE

### ETUDE DE L'ACTIVITE FONGICIDE

a) Essais Plasmopara viticola (Essai A).

Les jeunes plants de vigne issus de boutures (variété Grenache N, clone 70) sont cultivés en serre (température de jour : 30°C, température de nuit : 25°C) sur mélange terre/terreau/sable, (1/3-1/3-1/3). Deux jours avant l'essai, les plants sont transportés en chambre de culture (mêmes conditions de température, humidité : 60 % le jour, 80 % la nuit). Le produit est dissous dans la "matrice A" à une concentration de 500 ppm juste avant l'utilisation. Le traitement se fait par pulvérisation de la solution sur les feuilles jusqu'à rétention maximale. La contamination est faite avec une suspension de zoosporanges de Plasmopara viticola prélevés immédiatement avant l'essai (50.000 zoosporanges par ml). Les gouttes de suspension (20 microlitres) sont déposées à la face abaxiale des feuilles. Les plants sont ensuite maintenus pendant 24 heures dans une atmosphère saturée en humidité, puis ramenés à l'humidité de la chambre de culture (60 % le jour, 80 % la nuit).

La lecture s'effectue dix jours après la contamination, par mesure du développement des îlots de conidiophores à la surface abaxiale des feuilles. L'efficacité du produit est calculée par rapport à un témoin non traité.

b) Essais Erysiphe graminis hordei (Essai B).

Les grains d'orge (variété Pression) sont mis à germer dans le mélange terre-terreau-sable (1/3-1/3-1/3) et cultivés en serre. Les produits sont dissous dans la "matrice A" juste avant l'essai, à une concentration de 500 ppm. Le traitement s'effectue par pulvérisation de la solution de produit sur les plants d'orge âgés de dix jours, jusqu'à rétention maximale. La contamination par les conidies d'Erysiphe graminis hordei est faite trois jours après le traitement. Les plants sont maintenus en salle climatisée (température de jour : 23°C, température de nuit : 18°C). Sept jours après la contamination, on mesure l'étendue du feutrage conidien sur la première et la deuxième feuille de chaque plant. L'efficacité du produit est calculée par rapport à un témoin non traité.

c) Essais Puccinia recondita tritici (Essai C).

Les grains de blé (variété Festival) sont mis à germer dans le mélange terre-terreau-sable (1/3,1/3,1/3). Les plants sont cultivés en serre. Les produits sont dissous dans la "matrice A" juste avant l'essai, à une concentration de 500 ppm. Le traitement s'effectue par pulvérisation de la solution de produit sur les plants de blé âgés de neuf jours, jusqu'à rétention maximale. La contamination par les urédospores de Puccinia recondita tritici est faite le lendemain du traitement. Les plants sont maintenus en salle climatisée (température de jour : 22°C, température de nuit : 18°C). Sept jours après la contamination, on mesure la densité des spores sur les deux premières feuilles de chaque plant. L'efficacité du produit est calculée par rapport à un témoin non traité.

Résultats.

| EX. | Essai A | Essai B | | Essai C |
|---|---|---|---|---|
| | | 1ère F | 2ème F | |
| 1 | 90 | - | - | - |
| 2 | 90 | - | - | - |
| 3 | 100 | 80 | 30 | - |
| 4 | - | - | - | 70 |

F : Feuille ;
- : non déterminé.

Ces résultats montrent bien l'activité fongicide des produits des exemples 1 à 3 contre BOTRYTIS CINEREA sur vigne,

du produit de l'exemple 3 contre ERYSIPHE GRAMINIS HORDEI sur orge et du produit de l'exemple 4 sur PUCCINIA RECONDITA TRITICI sur blé.

| Composition de la matrice A : | |
|---|---|
| SOLVESSO 150 | 70,0 g |
| NAPSOL PM1 | 850,0 g |
| SURFAROX HRH 40C | 52,0 g |
| ECD 1604 | 28,0 g |
| | 1000,0 g |

## Revendications

**Revendications pour les Etats contractants suivants : AT, BE, CH, DE, DK, FR, GB, GR, IT, LI, LU, NL, PT, SE**

**1.** Les produits de formule (I) :

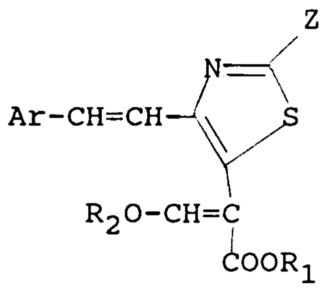

(I)

dans laquelle Ar représente un radical phényle non substitué ou substitué par un ou plusieurs radicaux identiques ou différents choisis parmi les atomes d'halogène, le radical méthylènedioxy, le radical phényle, le radical phénoxy, le radical trifluorométhyle, les radicaux alkyle linéaires ou ramifiés renfermant de un à six atomes de carbone, les radicaux alkyloxy linéaires ou ramifiés renfermant de un à six atomes de carbone ou les radicaux alkylthio linéaires ou ramifiés renfermant de un à six atomes de carbone, Z représente un atome d'hydrogène, un atome de chlore, un radical alkyle linéaire ou ramifié renfermant de un à six atomes de carbone,
$R_1$ et $R_2$ identiques ou différents représentent indépendamment l'un de l'autre, un radical alkyle linéaire ou ramifié renfermant de un à six atomes de carbone et les doubles liaisons exocycliques sont indépendamment l'une de l'autre en configuration (Z) ou en configuration (E).

**2.** Les produits de formule (I) telle que définie à la revendication 1 dans laquelle $R_1$ et $R_2$ représentent un radical méthyle.

**3.** Les produits de formule (I) telle que définie à la revendication 1 ou 2 dans laquelle Z représente un atome d'hydrogène, un atome de chlore ou un des radicaux choisis parmi les radicaux méthyle, éthyle, propyle et isopropyle.

**4.** Les produits dont les noms suivent :

- le 2-isopropyl alpha-[(Z)-méthoxy méthylène] 4-[(E)-styryl] 5-thiazolacétate de méthyle,
- le alpha-[(Z)-méthoxy méthylène] 2-méthyl 4-[(E)-styryl] 5-thiazolacétate de méthyle,
- le alpha-[(Z)-méthoxy méthylène] 4-[(E)-styryl] 5-thiazolacétate de méthyle.

**5.** Procédé de préparation des produits de formule (I) telle que définie à la revendication 1 caractérisé en ce qu'un ester de l'acide 5-Halo 4-oxo pentanoïque de formule (II) :

$$X\text{-}CH_2\text{-}CO\text{-}CH_2\text{-}CH_2\text{-}COOR_1 \qquad (II)$$

dans laquelle X représente un atome de chlore ou de brome et $R_1$ a la signification indiquée à la revendication 1, est traité par un phosphate de trialkyle de formule (III) :

$$(R_3O)_3P \qquad (III)$$

dans laquelle $R_3$ représente un radical méthyle ou un radical éthyle, pour former le phosphonate de formule (IV) :

$$(R_3O)_2\overset{\overset{O}{\uparrow}}{P}-CH_2-CO-CH_2-CH_2-COOR_1 \qquad (IV)$$

produit de formule (IV), que l'on traite en présence d'une base avec un aldéhyde de formule (V) :

$$Ar-CHO \qquad (V)$$

dans laquelle Ar a la signification indiquée à la revendication 1, pour obtenir le composé de formule (VI) :

$$Ar-CH=CH-CO-CH_2-CH_2-COOR_1 \qquad (VI)$$

ester de formule (VI), que l'on transforme par action d'un trialkyl chloro silane de formule (VII) :

$$R_4R_5R_6SiCl \qquad (VII)$$

dans laquelle $R_4$, $R_5$ et $R_6$ identiques ou différents, représentent un radical alkyle linéaire ou ramifié renfermant de un à quatre atomes de carbone, en un éther d'énol silylé de formule (VIII) :

$$Ar-CH=CH-C(OSiR_4R_5R_6)=CH-CH_2-COOR_1 \qquad (VIII)$$

produit de formule (VIII) qui, par bromation permet d'obtenir le dérivé de formule (IX) :

$$Ar-CH=CH-CO-CHBr-CH_2-COOR_1 \qquad (IX)$$

que l'on fait réagir soit avec le produit de formule (X) :

$$Z_1-CS-NH_2 \qquad (X)$$

dans laquelle $Z_1$ représente un atome d'hydrogène ou un radical alkyle linéaire ou ramifié, renfermant de un à six atomes de carbone, pour former un produit de formule $(XI)_1$ :

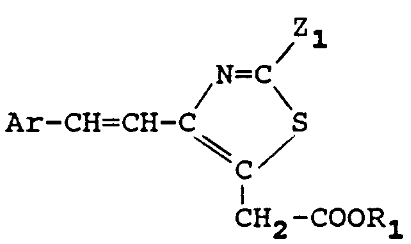

$(XI)_1$

soit avec un produit de formule (XII) :

$$Alc_1-O-CS-NH_2 \qquad (XII)$$

dans laquelle $Alc_1$ représente un radical alkyle renfermant de 1 à 4 atomes de carbone, pour former le dérivé de la 2-thiazolinone de formule (XIII) :

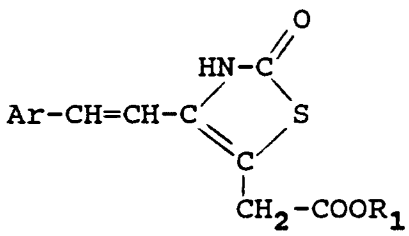

(XIII)

produit de formule (XIII) que l'on traite avec un réactif de chloration des fonctions carbonyle pour obtenir le produit de formule $(XI)_2$ :

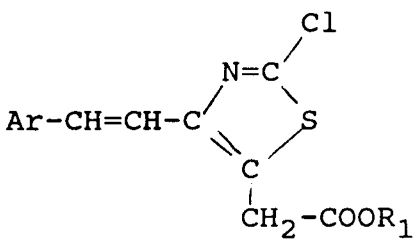

$(XI)_2$

produits de formules $(XI)_1$ ou $(XI)_2$, que l'on condense en présence d'une base avec un acétal du diméthyl formamide de formule (XVII) :

$$Me_2N\text{-}CH(OR_7)_2 \qquad (XVII)$$

dans laquelle $R_7$ représente un radical alkyle linéaire ou ramifié renfermant de un à six atomes de carbone, pour obtenir respectivement les produits de formules $(XVIII)_1$ et $(XVIII)_2$ :

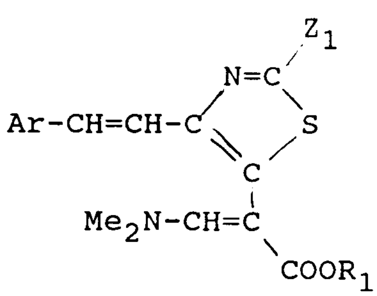

$(XVIII)_1$,

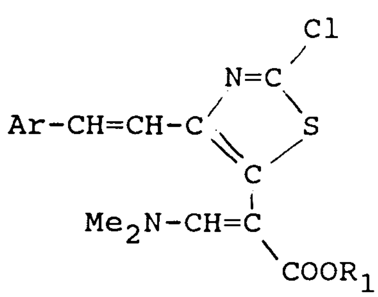

$(XVIII)_2$,

produits de formules $(XVIII)_1$ et $(XVIII)_2$, correspondant au produit de formule (XVIII) :

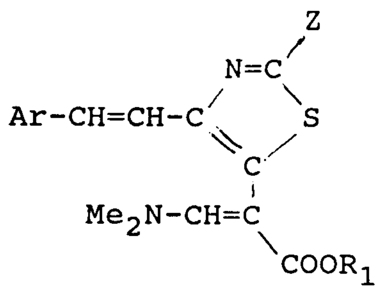

(XVIII)

dans laquelle Z, Ar et $R_1$ ont la signification indiquée à la revendication 1, produit de formule (XVIII) que l'on soumet à une réaction d'hydrolyse pour obtenir le composé de formule (XIX) :

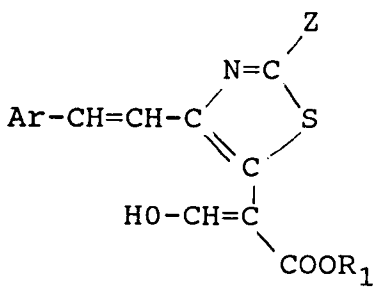

(XIX)

produit de formule (XIX) que l'on soumet à une réaction d'éthérification pour obtenir le produit de formule (I).

**6.** Utilisation à titre de fongicides des produits de formule (I) telle que définie à l'une quelconque des revendications 1 à 4.

**7.** Compositions fongicides comprenant à titre de principe actif, au moins un des produits de formule (I) telle que définie à l'une quelconque des revendications 1 à 4.

**8.** Les produits intermédiaires du procédé décrit à la revendication 5 de formules (IX), $(XI)_1$, $(XI)_2$, (XIII), (XVIII) et (XIX).

**Revendications pour l'Etat contractant suivant : ES**

**1.** Procédé pour préparer des produits de formule (I) :

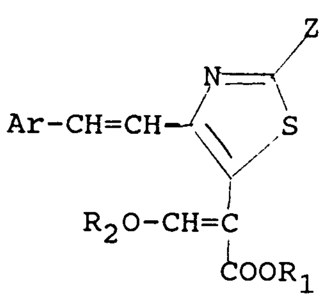

(I)

dans laquelle Ar représente un radical phényle non substitué ou substitué par un ou plusieurs radicaux identiques ou différents choisis parmi les atomes d'halogène, le radical méthylènedioxy, le radical phényle, le radical phénoxy, le radical trifluorométhyle, les radicaux alkyle linéaires ou ramifiés renfermant de un à six atomes de carbone, les radicaux alkyloxy linéaires ou ramifiés renfermant de un à six atomes de carbone ou les radicaux alkylthio linéaires ou ramifiés renfermant de un à six atomes de carbone, Z représente un atome d'hydrogène, un atome de chlore ou un radical alkyle linéaire ou ramifié renfermant de un à six atomes de carbone,

$R_1$ et $R_2$ identiques ou différents représentent indépendamment l'un de l'autre, un radical alkyle linéaire ou ramifié renfermant de un à six atomes de carbone et les doubles liaisons exocycliques sont indépendamment l'une de l'autre en configuration (Z) ou en configuration (E), caractérisé en ce qu'un ester de l'acide 5-Halo 4-oxo pentanoïque de formule (II) :

$$X\text{-}CH_2\text{-}CO\text{-}CH_2\text{-}CH_2\text{-}COOR_1 \qquad (II)$$

dans laquelle X représente un atome de chlore ou de brome et $R_1$ a la signification indiquée ci-dessus, est traité par un phosphate de trialkyle de formule (III) :

$$(R_3O)_3P \qquad (III)$$

dans laquelle $R_3$ représente un radical méthyle ou un radical éthyle, pour former le phosphonate de formule (IV) :

$$(R_3O)_2\overset{O}{\overset{\uparrow}{P}}-CH_2-CO-CH_2-CH_2-COOR_1 \qquad (IV)$$

produit de formule (IV), que l'on traite en présence d'une base avec un aldéhyde de formule (V) :

$$Ar\text{-}CHO \qquad (V)$$

dans laquelle Ar a la signification indiquée ci-dessus, pour obtenir le composé de formule (VI) :

$$Ar\text{-}CH{=}CH\text{-}CO\text{-}CH_2\text{-}CH_2\text{-}COOR_1 \qquad (VI)$$

ester de formule (VI), que l'on transforme par action d'un trialkyl chloro silane de formule (VII) :

$$R_4R_5R_6SiCl \qquad (VII)$$

dans laquelle $R_4$, $R_5$ et $R_6$ identiques ou différents, représentent un radical alkyle linéaire ou ramifié renfermant de un à quatre atomes de carbone, en un éther d'énol silylé de formule (VIII) :

$$Ar\text{-}CH{=}CH\text{-}C(OSiR_4R_5R_6){=}CH\text{-}CH_2\text{-}COOR_1 \qquad (VIII)$$

produit de formule (VIII) qui, par bromation permet d'obtenir le dérivé de formule (IX) :

$$Ar\text{-}CH{=}CH\text{-}CO\text{-}CHBr\text{-}CH_2\text{-}COOR_1 \qquad (IX)$$

que l'on fait réagir soit avec le produit de formule (X) :

$$Z_1\text{-}CS\text{-}NH_2 \qquad (X)$$

dans laquelle $Z_1$ représente un atome d'hydrogène ou un radical alkyle linéaire ou ramifié, renfermant de un à six atomes de carbone, pour former un produit de formule $(XI)_1$ :

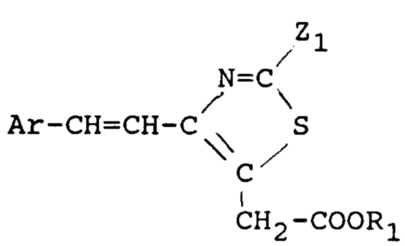

$(XI)_1$

<u>soit</u> avec un produit de formule (XII) :

$Alc_1$-O-CS-$NH_2$ (XII)

dans laquelle $Alc_1$ représente un radical alkyle renfermant de 1 à 4 atomes de carbone, pour former le dérivé de la 2-thiazolinone de formule (XIII) :

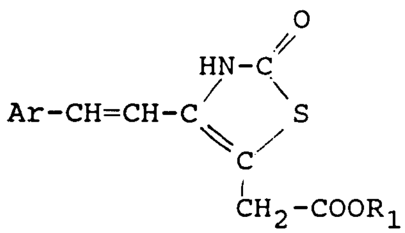

(XIII)

produit de formule (XIII) que l'on traite avec un réactif de chloration des fonctions carbonyle pour obtenir le produit de formule $(XI)_2$ :

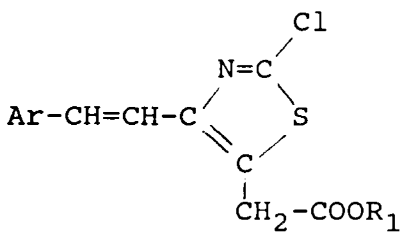

$(XI)_2$

produits de formules $(XI)_1$ ou $(XI)_2$, que l'on condense en présence d'une base avec un acétal du diméthyl formamide de formule (XVII) :

$Me_2N$-$CH(OR_7)_2$ (XVII)

dans laquelle $R_7$ représente un radical alkyle linéaire ou ramifié renfermant de un à six atomes de carbone, pour obtenir respectivement les produits de formules $(XVIII)_1$ et $(XVIII)_2$ :

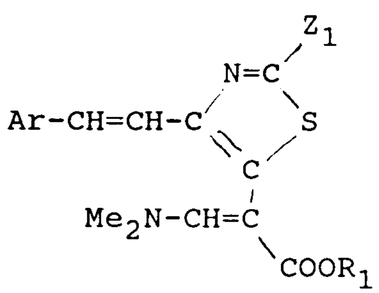

$(XVIII)_1$,

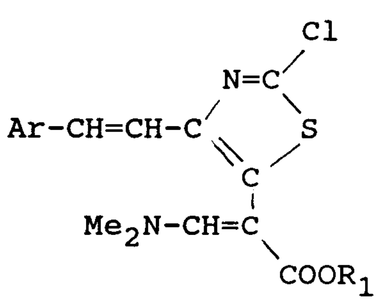

$(XVIII)_2$,

produits de formules $(XVIII)_1$ et $(XVIII)_2$ correspondant au produit de formule (XVIII) :

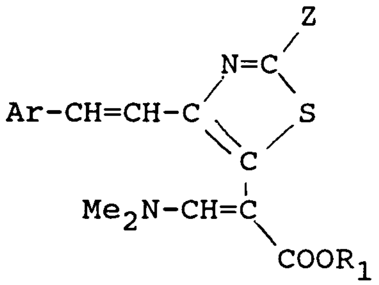

(XVIII)

dans laquelle Z, Ar et $R_1$ ont la signification indiquée ci-dessus, produit de formule (XVIII) que l'on soumet à une réaction d'hydrolyse pour obtenir le composé de formule (XIX) :

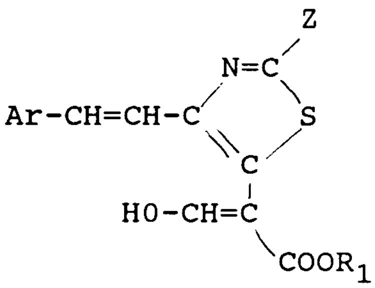

(XIX)

produit de formule (XIX) que l'on soumet à une réaction d'éthérification pour obtenir le produit de formule (I).

**2.** Procédé selon la revendication 1, caractérisé en ce que l'on utilise au départ un produit de formule (II) dans laquelle $R_1$ représente un radical méthyle et en ce que l'éthérification du produit de formule (XIX) est effectuée au moyen d'un halogénure de méthyle.

**3.** Procédé selon la revendication 1 ou 2, caractérisé en ce que soit l'on traite un produit de formule (XIII) par un agent de chloration, soit l'on traite un produit de formule (IX) par un réactif de formule (X) ou (XIV) choisi de manière telle que l'on prépare un produit de formule $(XVIII_1)$ dans lequel $Z_1$ ou $Z_3$ représente un atome d'hydrogène, un radical méthyle, éthyle, propyle ou isopropyle

**Claims**

**Claims for the following Contracting States : AT, BE, CH, DE, DK, FR, GB, GR, IT, LI, LU, NL, PT, SE**

**1.** The products of formula (I):

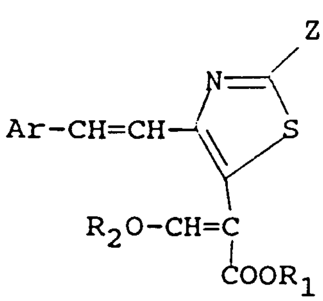

(I)

in which Ar represents a phenyl radical non-substituted or substituted by one or more identical or different radicals chosen from halogen atoms, the methylenedioxy radical, the phenyl radical, the phenoxy radical, the trifluoromethyl

radical, linear or branched alkyl radicals containing 1 to 6 carbon atoms, linear or branched alkyloxy radicals containing 1 to 6 carbon atoms or linear or branched alkylthio radicals containing 1 to 6 carbon atoms, Z represents a hydrogen atom, a chlorine atom, a linear or branched alkyl radical containing 1 to 6 carbon atoms,
$R_1$ and $R_2$, identical or different, represent independently of each other a linear or branched alkyl radical containing 1 to 6 carbon atoms and the exocyclic double bonds are independently of each other in (Z) configuration or in (E) configuration.

**2.** The products of formula (I) as defined in claim 1 in which $R_1$ and $R_2$ represent a methyl radical.

**3.** The products of formula (I) as defined in claim 1 or 2 in which Z represents a hydrogen atom, a chlorine atom or one of the radicals chosen from methyl, ethyl, propyl and isopropyl.

**4.** The products the names of which follow:

- methyl 2-isopropyl-alpha-[(Z)-methoxy methylene]-4-[(E)-styryl]-5-thiazolacetate,
- methyl alpha-[(Z)-methoxy methylene]-2-methyl-4-[(E)-styryl]-5-thiazolacetate,
- methyl alpha-[(Z)-methoxy methylene]-4-[(E)-styryl]-5-thiazolacetate.

**5.** The preparation process for products of formula (I) as defined in claim 1 characterized in that an ester of 5-Halo-4-oxo pentanoic acid of formula (II):

$$X\text{-}CH_2\text{-}CO\text{-}CH_2CH_2\text{-}COOR_1 \qquad (II)$$

in which X represents a chlorine or bromine atom and $R_1$ has the meaning indicated in claim 1, is treated with a trialkyl phosphate of formula (III):

$$(R_3O)_3P \qquad (III)$$

in which $R_3$ represents a methyl radical or an ethyl radical, in order to form the phosphonate of formula (IV):

$$(R_3O)_2\overset{O}{\overset{\uparrow}{P}}\text{-}CH_2\text{-}CO\text{-}CH_2\text{-}CH_2\text{-}COOR_1 \qquad (IV)$$

which product of formula (IV) is treated in the presence of a base with an aldehyde of formula (V):

$$Ar\text{-}CHO \qquad (V)$$

in which Ar has the meaning indicated in claim 1, in order to obtain the compound of formula (VI):

$$Ar\text{-}CH{=}CH\text{-}CO\text{-}CH_2\text{-}CH_2\text{-}COOR_1 \qquad (VI)$$

which ester of formula (VI) is converted by the action of a trialkyl chlorosilane of formula (VII):

$$R_4R_5R_6SiCl \qquad (VII)$$

in which $R_4$, $R_5$ and $R_6$, identical or different, represent a linear or branched alkyl radical containing 1 to 4 carbon atoms, into a silylated enol ether of formula (VIII):

$$Ar\text{-}CH{=}CH\text{-}C(OSiR_4R_5R_6){=}CH\text{-}CH_2\text{-}CCOR_1 \qquad (VIII)$$

which product of formula (VIII), by bromination, enables the derivative of formula (IX):

$$Ar\text{-}CH{=}CH\text{-}CO\text{-}CHBr\text{-}CH_2\text{-}COOR_1 \qquad (IX)$$

to be obtained, which is reacted <u>either</u> with the product of formula (X):

$Z_1$-CS-$NH_2$ (X)

in which $Z_1$ represents a hydrogen atom or a linear or branched alkyl radical, containing 1 to 6 carbon atoms, in order to form a product of formula $(XI)_1$:

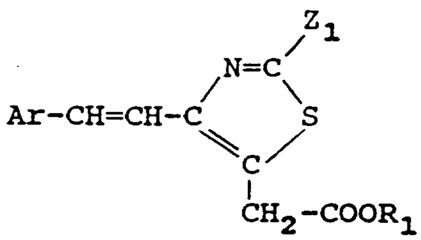

$(XI)_1$

or with a product of formula (XII):

$Alk_1$-O-CS-$NH_2$ (XII)

in which $Alk_1$ represents an alkyl radical containing 1 to 4 carbon atoms, in order to form the 2-thiazolinone derivative of formula (XIII):

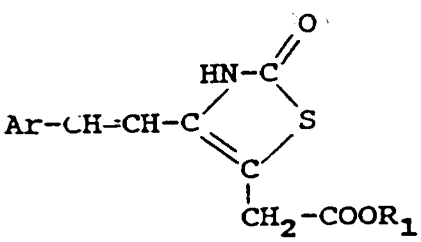

(XIII)

which product of formula (XIII) is treated with a chlorination reagent of the carbonyl functions in order to obtain the product of formula $(XI)_2$:

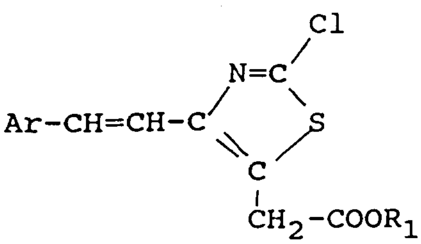

$(XI)_2$

which products of formulae $(XI)_1$ or $(XI)_2$, are condensed in the presence of a base with a dimethyl formamide acetal of formula (XVII):

$Me_2N$-$CH(OR_7)_2$ (XVII)

in which $R_7$ represents a linear or branched alkyl radical containing 1 to 6 carbon atoms, in order to obtain respectively the products of formulae $(XVIII)_1$ and $(XVIII)_2$ respectively:

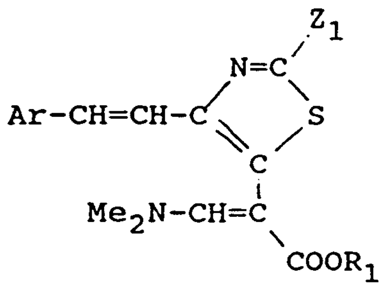

$(XVIII)_1$

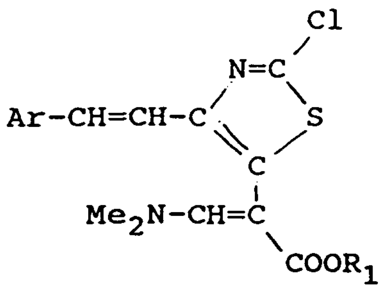

$(XVIII)_2$

which products of formulae $(XVIII)_1$ and $(XVIII)_2$, correspond to the product of formula (XVIII):

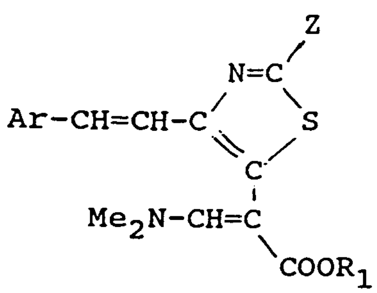

(XVIII)

in which Z, Ar and $R_1$ have the meaning indicated in claim 1, which product of formula (XVIII) is subjected to a hydrolysis reaction in order to obtain the compound of formula (XIX):

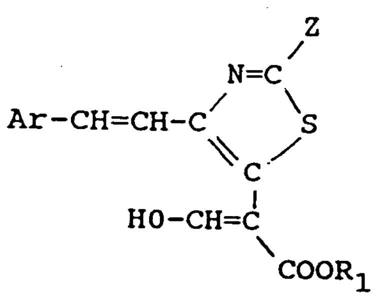

(XIX)

which product of formula (XIX) is subjected to an etherification reaction in order to obtain the product of formula (I).

6. Use as fungicides of the products of formula (I) as defined in any one of claims 1 to 4.

7. Fungicide compositions containing as active ingredient at least one of the products of formula (I) as defined in any one of claims 1 to 4.

8. Intermediate products of the process described in claim 5 of formulae (IX), $(XI)_1$, $(XI)_2$, (XIII), (XVIII) and (XIX).

**Claims for the following Contracting State : ES**

1. Preparation process for the products of formula (I):

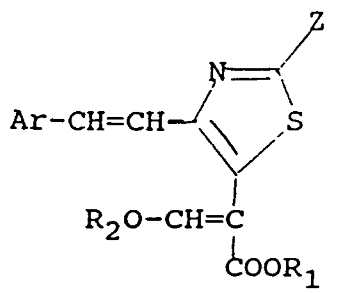

(I)

in which Ar represents a phenyl radical non-substituted or substituted by one or more identical or different radicals chosen from halogen atoms, the methylenedioxy radical, the phenyl radical, the phenoxy radical, the trifluoromethyl radical, linear or branched alkyl radicals containing 1 to 6 carbon atoms, linear or branched alkyloxy radicals containing 1 to 6 carbon atoms or linear or branched alkylthio radicals containing 1 to 6 carbon atoms, Z represents a hydrogen atom, a chlorine atom, a linear or branched alkyl radical containing 1 to 6 carbon atoms,
$R_1$ and $R_2$, identical or different, represent independently of each other a linear or branched alkyl radical containing 1 to 6 carbon atoms and the exocyclic double bonds are independently of each other in (Z) configuration or in (E) configuration characterized in that an ester of 5-Halo-4-oxo pentanoic acid of formula (II):

$$X\text{-}CH_2\text{-}CO\text{-}CH_2CH_2\text{-}COOR_1 \qquad (II)$$

in which X represents a chlorine or bromine atom and $R_1$ has the meaning indicated above, is treated with a trialkyl phosphate of formula (III):

$$(R_3O)_3P \qquad (III)$$

in which $R_3$ represents a methyl radical or an ethyl radical, in order to form the phosphonate of formula (IV):

$$(R_3O)_2\overset{O}{\overset{\uparrow}{P}}-CH_2-CO-CH_2-CH_2-COOR_1 \qquad (IV)$$

which product of formula (IV) is treated in the presence of a base with an aldehyde of formula (V):

$$Ar\text{-}CHO \qquad (V)$$

in which Ar has the meaning indicated above, in order to obtain the compound of formula (VI):

$$Ar\text{-}CH{=}CH\text{-}CO\text{-}CH_2\text{-}CH_2\text{-}COOR_1 \qquad (VI)$$

which ester of formula (VI) is converted by the action of a trialkyl chlorosilane of formula (VII):

$$R_4R_5R_6SiCl \qquad (VII)$$

in which $R_4$, $R_5$ and $R_6$, identical or different, represent a linear or branched alkyl radical containing 1 to 4 carbon atoms, into a silylated enol ether of formula (VIII):

$$Ar\text{-}CH{=}CH\text{-}C(OSiR_4R_5R_6){=}CH\text{-}CH_2\text{-}COOR_1 \quad (VIII)$$

which product of formula (VIII), by bromination, enables the derivative of formula (IX):

$$Ar\text{-}CH{=}CH\text{-}CO\text{-}CHBr\text{-}CH_2\text{-}COOR_1 \quad (IX)$$

to be obtained, which is reacted either with the product of formula (X):

$$Z_1\text{-}CS\text{-}NH_2 \quad (X)$$

in which $Z_1$ represents a hydrogen atom or a linear or branched alkyl radical, containing 1 to 6 carbon atoms, in order to form a product of formula $(XI)_1$:

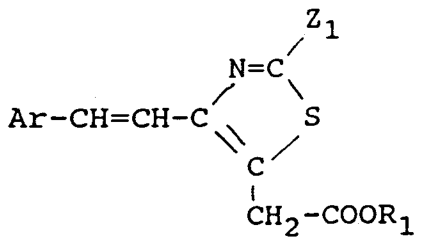

$(XI)_1$

or with a product of formula (XII):

$$Alk_1\text{-}O\text{-}CS\text{-}NH_2 \quad (XII)$$

in which $Alk_1$ represents an alkyl radical containing 1 to 4 carbon atoms, in order to form the 2-thiazolinone derivative of formula (XIII):

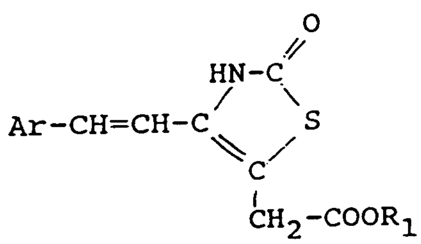

(XIII)

which product of formula (XIII) is treated with a chlorination reagent of the carbonyl functions in order to obtain the product of formula $(XI)_2$:

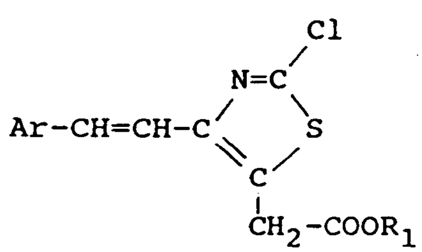

$(XI)_2$

which products of formulae $(XI)_1$ or $(XI)_2$, are condensed in the presence of a base with a dimethyl formamide acetal of formula (XVII):

$$Me_2N\text{-}CH(OR_7)_2 \quad (XVII)$$

in which $R_7$ represents a linear or branched alkyl radical containing 1 to 6 carbon atoms, in order to obtain respectively the products of formulae $(XVIII)_1$ and $(XVIII)_2$ respectively:

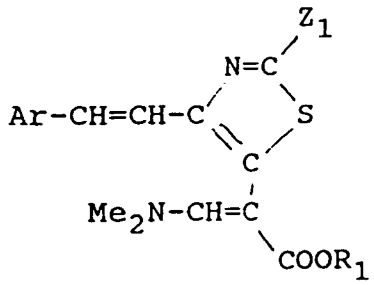

$(XVIII)_1$

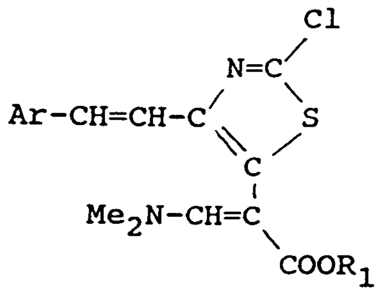

$(XVIII)_2$

which products of formulae $(XVIII)_1$ and $(XVIII)_2$, correspond to the product of formula (XVIII):

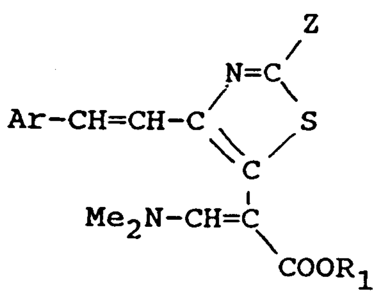

(XVIII)

in which Z, Ar and $R_1$ have the meaning indicated above, which product of formula (XVIII) is subjected to a hydrolysis reaction in order to obtain the compound of formula (XIX):

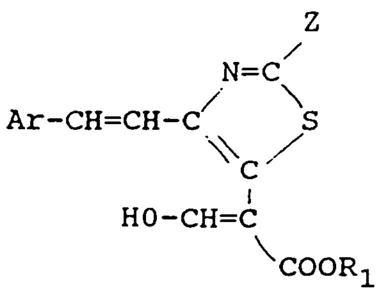

(XIX)

which product of formula (XIX) is subjected to an etherification reaction in order to obtain the product of formula (I).

2. Process according to claim 1, characterized in that a product of formula (II) is used at the start in which $R_1$ represents a methyl radical and in that the etherification of the product of formula (XIX) is carried out by means of a methyl halide.

3. Process according to claim 1 or 2, characterized in that either a product of formula (XIII) is treated with a chlorination agent, or a product of formula (IX) is treated with a reagent of formula (X) or (XIV) chosen so as to prepare a product of formula ($XVIII_1$) in which $Z_1$ or $Z_3$ represents a hydrogen atom, a methyl, ethyl, propyl or isopropyl radical.

**Patentansprüche**

**Patentansprüche für folgende Vertragsstaaten : AT, BE, CH, DE, DK, FR, GB, GR, IT, LI, LU, NL, PT, SE**

1. Produkte der Formel (I)

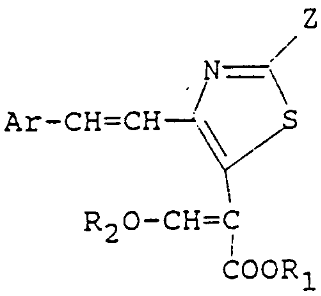

(I)

worin Ar einen Phenylrest bedeutet, der nicht substituiert ist oder substituiert ist durch einen oder mehrere gleiche oder verschiedene Reste, ausgewählt unter den Halogenatomen, dem Methylendioxyrest, dem Phenylrest, dem Phenoxyrest, dem Trifluormethylrest, den linearen oder verzweigten Alkylresten mit 1 bis 6 Kohlenstoffatomen, den linearen oder verzweigten Alkoxyresten mit 1 bis 6 Kohlenstoffatomen oder den linearen oder verzweigten Alkylthioresten mit 1 bis 6 Kohlenstoffatomen, Z ein Wasserstoffatom, ein Chloratom oder einen linearen oder verzweigten Alkylrest mit 1 bis 6 Kohlenstoffatomen bedeutet, $R_1$ und $R_2$, identisch oder verschieden, unabhängig voneinander einen linearen oder verzweigten Alkylrest mit 1 bis 6 Kohlenstoffatomen bedeuten und die exocyclischen Doppelbindungen voneinander unabhängig in (Z)-Konfiguration oder in (E)-Konfiguration vorliegen.

2. Produkte der Formel (I), wie in Anspruch 1 definiert, worin $R_1$ und $R_2$ einen Methylrest bedeuten.

3. Produkte der Formel (I), wie in Anspruch 1 oder 2 definiert, worin Z ein Wasserstoffatom, ein Chloratom oder einen der Reste, ausgewählt unter den Methyl-, Ethyl-, Propyl- und Isopropylresten, bedeutet.

4. Produkte mit den folgenden Bezeichnungen:
   2-Isopropyl-α-[(Z)-methoxy-methylen]-4-[(E)-Styryl]-5-thiazol-methylacetat,
   α-[(Z)-Methoxy-methylen]-2-methyl-4-[(E)-styryl]-5-thiazol-methylacetat,
   α-[(Z)-Methoxy-methylen]-4-[(E)-styryl]-5-thiazol-methylacetat.

5. Verfahren zur Herstellung der Produkte der Formel (I), wie in Anspruch 1 definiert, dadurch gekennzeichnet, daß man einen Ester der 5-Halo-4-oxo-pentansäure der Formel (II)

$$X\text{-}CH_2\text{-}CO\text{-}CH_2\text{-}CH_2\text{-}COOR_1 \qquad (II)$$

worin X ein Chlor- oder Bromatom bedeutet und $R_1$ die in Anspruch 1 angegebene Bedeutung besitzt, mit einem Trialkylphosphat der Formel (III)

$$(R_3O)_3P \qquad (III)$$

worin $R_3$ einen Methylrest oder einen Ethylrest bedeutet, behandelt, um das Phosphonat der Formel (IV)

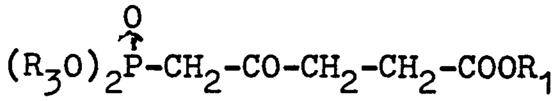

(IV)

zu bilden, das Produkt der Formel (IV) in Gegenwart einer Base mit einem Aldehyd der Formel (V)

$$Ar\text{-}CHO \qquad (V)$$

worin Ar wie in Anspruch 1 definiert ist, behandelt, um zu der Verbindung der Formel (VI)

$$Ar\text{-}CH{=}CH\text{-}CO\text{-}CH_2\text{-}CH_2\text{-}COOR_1 \qquad (VI)$$

zu gelangen, den Ester der Formel (VI) durch Einwirken von Trialkylchlorsilan der Formel (VII)

$$R_4R_5R_6SiCl \qquad (VII)$$

worin $R_4$, $R_5$ und $R_6$, identisch oder verschieden, einen linearen oder verzweigten Alkylrest mit 1 bis 4 Kohlenstoffatomen bedeuten, in einen silylierten Enolether der Formel (VIII)

$$Ar\text{-}CH{=}CH\text{-}C(OSiR_4R_5R_6){=}CH\text{-}CH_2\text{-}COOR_1 \qquad (VIII)$$

überführt, dieses Produkt der Formel (VIII) durch Bromierung das Derivat der Formel (IX) erzielen läßt,

$$Ar\text{-}CH{=}CH\text{-}CO\text{-}CHBr\text{-}CH_2\text{-}COOR_1 \qquad (IX)$$

welches man entweder mit dem Produkt der Formel (X)

$$Z_1\text{-}CS\text{-}NH_2 \qquad (X)$$

worin $Z_1$ ein Wasserstoffatom oder einen linearen oder verzweigten Alkylrest mit 1 bis 6 Kohlenstoffatomen bedeutet, umsetzt, um ein Produkt der Formel $(XI)_1$

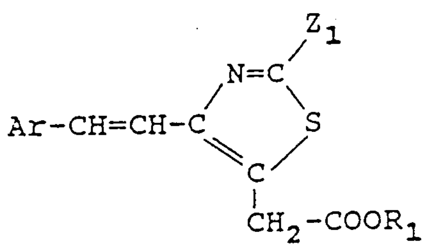

$(XI)_1$

zu bilden, oder mit einem Produkt der Formel (XII)

$$Alc_1\text{-}O\text{-}CS\text{-}NH_2 \qquad (XII)$$

worin $Alc_1$ einen Alkylrest mit 1 bis 4 Kohlenstoffatomen bedeutet, umsetzt, um das 2-Thiazolinonderivat der Formel (XIII)

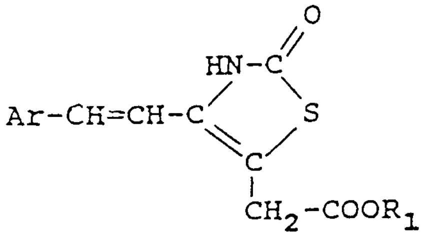

(XIII)

zu bilden, das Produkt der Formel (XIII) mit einem Chlorierungsreagens der Carbonylfunktionen behandelt, um zu dem Produkt der Formel $(XI)_2$ zu gelangen,

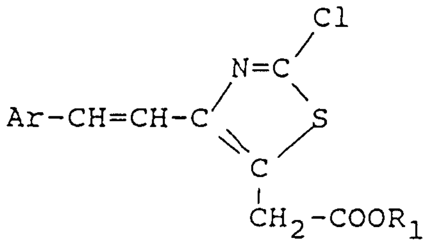

$(XI)_2$

die Produkte der Formeln $(XI)_1$ oder $(XI)_2$ in Anwesenheit einer Base mit einem Acetal des Dimethylformamids der Formel (XVII)

$$Me_2N\text{-}CH(OR_7)_2 \quad \text{(XVII)}$$

worin $R_7$ einen linearen oder verzweigten Alkylrest mit 1 bis 6 Kohlenstoffatomen bedeutet, kondensiert, um zu den Produkten der Formeln $(XVIII)_1$ bzw. $(XVIII)_2$ zu gelangen,

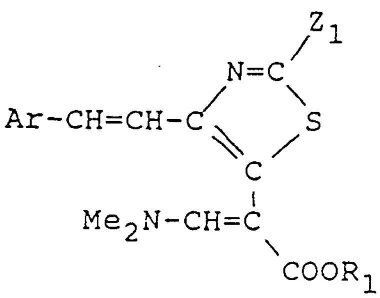

$(XVIII)_1$,

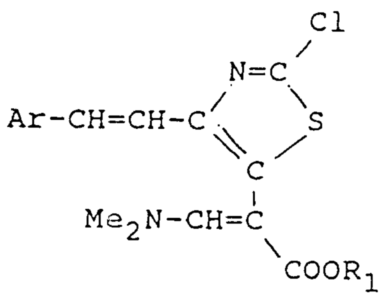

$(XVIII)_2$,

wobei die Produkte der Formeln $(XVIII)_1$ und $(XVIII)_2$ dem Produkt der Formel (XVIII)

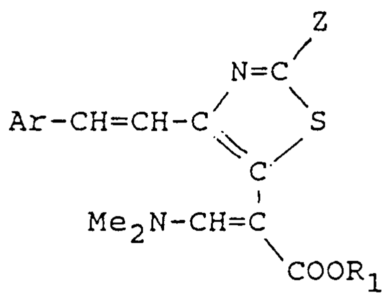

(XVIII)

entsprechen, worin Z, Ar und $R_1$ die in Anspruch 1 angegebene Bedeutung besitzen, das Produkt der Formel (XVIII) einer Hydrolysereaktion unterzieht, um zu der Verbindung der Formel (XIX)

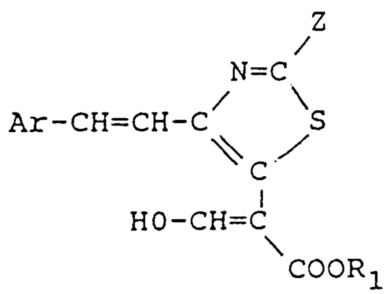

(XIX)

zu gelangen, das Produkt der Formel (XIX) einer Veretherungsreaktion unterzieht, um zu dem Produkt der Formel (I) zu gelangen.

6. Verwendung der Produkte der Formel (I), wie in einem der Ansprüche 1 bis 4 definiert, als Fungizide.

7. Fungizide Zusammensetzungen, umfassend als Wirkstoff zumindest eines der Produkte der Formel (I), wie in einem der Ansprüche 1 bis 4 definiert.

8. Zwischenprodukte des in Anspruch 5 beschriebenen Verfahrens der Formeln (IX), $(XI)_1$, $(XI)_2$, (XIII), (XVIII) und (XIX).

**Patentansprüche für folgenden Vertragsstaat : ES**

1. Verfahren zur Herstellung der Produkte der Formel (I)

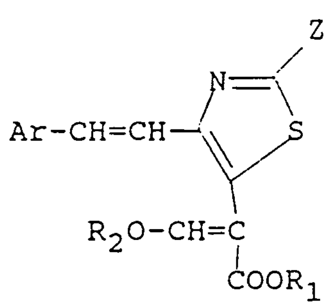

(I)

worin Ar einen Phenylrest bedeutet, der nicht substituiert ist oder substituiert ist durch einen oder mehrere gleiche oder verschiedene Reste, ausgewählt unter den Halogenatomen, dem Methylendioxyrest, dem Phenylrest, dem Phenoxyrest, dem Trifluormethylrest, den linearen oder verzweigten Alkylresten mit 1 bis 6 Kohlenstoffatomen, den

linearen oder verzweigten Alkoxyresten mit 1 bis 6 Kohlenstoffatomen oder den linearen oder verzweigten Alkylthioresten mit 1 bis 6 Kohlenstoffatomen, Z ein Wasserstoffatom, ein Chloratom oder einen linearen oder verzweigten Alkylrest mit 1 bis 6 Kohlenstoffatomen bedeutet, $R_1$ und $R_2$, identisch oder verschieden, unabhängig voneinander einen linearen oder verzweigten Alkylrest mit 1 bis 6 Kohlenstoffatomen bedeuten und die exocyclischen Doppelbindungen voneinander unabhängig in (Z)-Konfiguration oder in (E)-Konfiguration vorliegen,
dadurch gekennzeichnet, daß man einen Ester der 5-Halo-4-oxopentansäure der Formel (II)

$$X\text{-}CH_2\text{-}CO\text{-}CH_2\text{-}CH_2\text{-}COOR_1 \qquad (II)$$

worin X ein Chlor- oder Bromatom bedeutet und $R_1$ wie vorstehend definiert ist, mit einem Trialkylphosphat der Formel (III)

$$(R_3O)_3P \qquad (III)$$

worin $R_3$ einen Methylrest oder einen Ethylrest bedeutet, behandelt, um das Phosphonat der Formel (IV)

$$(R_3O)_2\overset{\overset{O}{\uparrow}}{P}\text{-}CH_2\text{-}CO\text{-}CH_2\text{-}CH_2\text{-}COOR_1 \qquad (IV)$$

zu bilden, das Produkt der Formel (IV) in Gegenwart einer Base mit einem Aldehyd der Formel (V)

$$Ar\text{-}CHO \qquad (V)$$

worin Ar wie vorstehend definiert ist, behandelt, um zu der Verbindung der Formel (VI)

$$Ar\text{-}CH{=}CH\text{-}CO\text{-}CH_2\text{-}CH_2\text{-}COOR_1 \qquad (VI)$$

zu gelangen, den Ester der Formel (VI) durch Einwirken von Trialkylchlorsilan der Formel (VII)

$$R_4R_5R_6SiCl \qquad (VII)$$

worin $R_4$, $R_5$ und $R_6$, identisch oder verschieden, einen linearen oder verzweigten Alkylrest mit 1 bis 4 Kohlenstoffatomen bedeuten, in einen silylierten Enolether der Formel (VIII)

$$Ar\text{-}CH{=}CH\text{-}C(OSiR_4R_5R_6){=}CH\text{-}CH_2\text{-}COOR_1 \qquad (VIII)$$

überführt, dieses Produkt der Formel (VIII) durch Bromierung das Derivat der Formel (IX) erzielen läßt,

$$Ar\text{-}CH{=}CH\text{-}CO\text{-}CHBr\text{-}CH_2\text{-}COOR_1 \qquad (IX)$$

welches man entweder mit dem Produkt der Formel (X)

$$Z_1\text{-}CS\text{-}NH_2 \qquad (X)$$

worin $Z_1$ ein Wasserstoffatom oder einen linearen oder verzweigten Alkylrest mit 1 bis 6 Kohlenstoffatomen bedeutet, umsetzt, um ein Produkt der Formel $(XI)_1$

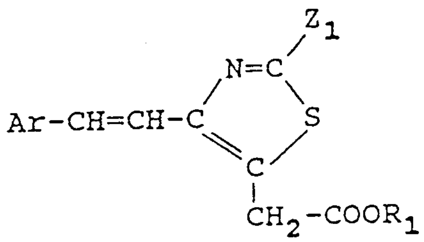

$(XI)_1$

zu bilden, oder mit einem Produkt der Formel (XII)

$Alc_1$-O-CS-$NH_2$ (XII)

worin $Alc_1$ einen Alkylrest mit 1 bis 4 Kohlenstoffatomen bedeutet, umsetzt, um das 2-Thiazolinonderivat der Formel (XIII)

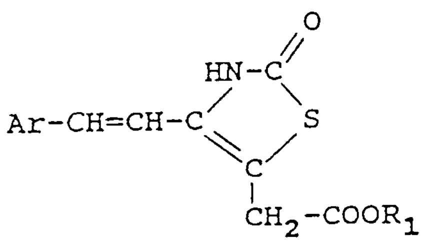

(XIII)

zu bilden, das Produkt der Formel (XIII) mit einem Chlorierungsreagens der Carbonylfunktionen behandelt, um zu dem Produkt der Formel $(XI)_2$ zu gelangen,

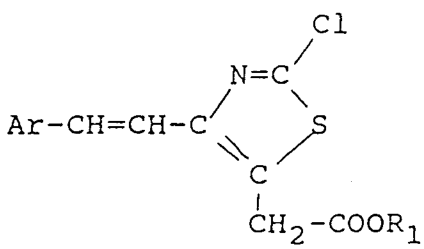

$(XI)_2$

die Produkte der Formeln $(XI)_1$ oder $(XI)_2$ in Anwesenheit einer Base mit einem Acetal des Dimethylformamids der Formel (XVII)

$Me_2N$-CH$(OR_7)_2$ (XVII)

worin $R_7$ einen linearen oder verzweigten Alkylrest mit 1 bis 6 Kohlenstoffatomen bedeutet, kondensiert, um zu den Produkten der Formeln $(XVIII)_1$ bzw. $(XVIII)_2$ zu gelangen,

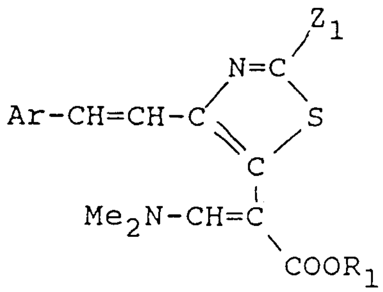

$(XVIII)_1$,

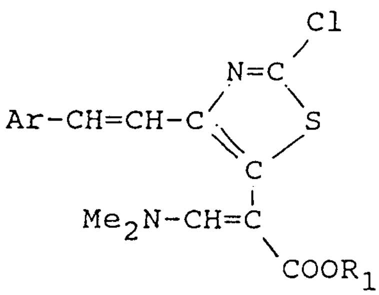

$(XVIII)_2$,

wobei die Produkte der Formeln $(XVIII)_1$ und $(XVIII)_2$ dem Produkt der Formel (XVIII)

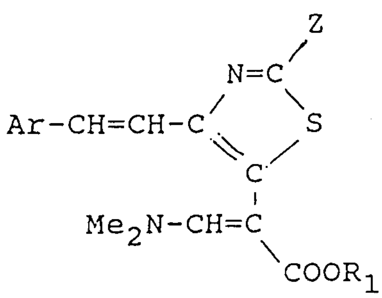

(XVIII)

entsprechen, worin Z, Ar und $R_1$ die vorstehend angegebene Bedeutung besitzen, das Produkt der Formel (XVIII) einer Hydrolysereaktion unterzieht, um zu der Verbindung der Formel (XIX)

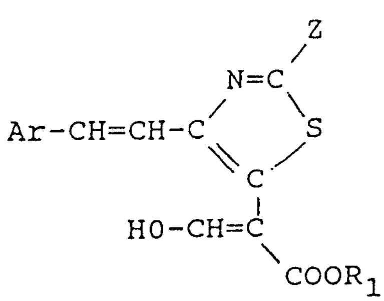

(XIX)

zu gelangen, das Produkt der Formel (XIX) einer Veretherungsreaktion unterzieht, um zu dem Produkt der Formel (I) zu gelangen.

2. Verfahren gemäß Anspruch 1, dadurch gekennzeichnet, daß man von einem Produkt der Formel (II) ausgeht, worin $R_1$ einen Methylrest bedeutet, und daß die Veretherung des Produkts der Formel (XIX) mit Hilfe von Methylhalogenid durchgeführt wird.

3. Verfahren gemäß Anspruch 1 oder 2, dadurch gekennzeichnet, daß man entweder ein Produkt der Formel (XIII) mit einem Chlorierungsreagens behandelt oder ein Produkt der Formel (IX) mit einem Reagens der Formel (X) oder (XIV) behandelt, das derart ausgewählt wird, daß man ein Produkt der Formel $(XVIII)_1$ herstellt, worin $Z_1$ oder $Z_3$ ein Wasserstoffatom, einen Methyl-, Ethyl-, Propyl- oder Isopropylrest bedeutet.